# EUROPEAN PATENT APPLICATION

(11) **EP 4 162 952 A1**
(43) Date of publication of application: **12.04.2023**
(21) Application number: 21797818.8
(22) Date of filing: 28.04.2021
(51) Int. Cl.: A61K 45/00, A61K 31/138, A61K 31/41, A61K 31/4439, A61K 31/4535, A61K 31/496

(54) **PROPHYLACTIC OR THERAPEUTIC AGENT FOR RNA VIRUS-RELATED DISEASES**

(30) Priority: 30.04.2020 US 202063017677 P; 11.12.2020 US 202063124098 P
(71) Applicant: Kyoto University, Kyoto-shi, Kyoto 606-8501 (JP); NAGASAKI UNIVERSITY, Nagasaki 852-8521 (JP)
(72) Inventor: INOUE Haruhisa, Kyoto-shi, Kyoto 606-8501 (JP); IMAMURA Keiko, Kyoto-shi, Kyoto 606-8501 (JP); YASUDA Jiro, Nagasaki-shi, Nagasaki 852-8521 (JP); SAKURAI Yasuteru, Nagasaki-shi, Nagasaki 852-8521 (JP)
(74) Representative: Hoffmann Eitle
(86) International application number: PCT/JP2021/017002
(87) International publication number: WO 2021/221111

(57) **Abstract**

A a prophylactic or therapeutic agent for RNA virus-related diseases is provided, the prophylactic or therapeutic agent containing, as an active ingredient, at least one compound selected from the group consisting of a selective estrogen receptor modulator, an anti-tuberculosis drug, a CysLT1 receptor antagonist, a peroxisome proliferator-activated receptor γ (PPARy) agonist, an arachidonate 5-lipoxygenase (5-LOX) inhibitor, a derivative thereof, a pharmaceutically acceptable salt thereof, and a pharmaceutically acceptable solvate thereof.

## Description

### Field of the Invention

The present invention relates to a prophylactic or therapeutic agent for RNA virus-related diseases. Priority is claimed on the specification of United States Patent Application, Publication No. 63/017,677, provisionally filed in the United States on April 30, 2020, and the specification of United States Patent Application, Publication No. 63/124,098, provisionally filed in the United States on December 11, 2020, the contents of which are incorporated herein by reference.

### Description of Related Art

The emergence of severe acute respiratory syndrome coronavirus 2 (SARS-CoV-2) has caused a pandemic due to efficient human-to-human infection and high pathogenicity and has become a public health threat (for example, Non-Patent Document 1). SARS-CoV-2 is a virus belonging to Coronaviridae and is an enveloped virus having a single-stranded positive sense RNA genome.

The causative virus of emerging viral diseases such as severe acute respiratory syndrome (SARS) and Middle East respiratory syndrome (MERS) also belongs to Coronaviridae. In addition, the causative virus of Ebola virus disease and Marburg virus disease, which have a high mortality rate, belongs to Filoviridae, which is an enveloped virus having a single-stranded negative sense RNA genome.

### Citation List

### [Non-Patent Document]

[Non-Patent Document 1]
R. T. Gandhi, et al., Mild or Moderate Covid-19, N Engl J Med, 383, 1757-1766, 2020.

### Summary of the Invention

### Technical Problem

In addition to the coronavirus and the filovirus, a large number of RNA viruses have become a threat to emerging and re-emerging infectious diseases, and thus it is very important to find a prophylactic or therapeutic agent for RNA virus-related diseases. An object of the present invention is to provide a prophylactic or therapeutic agent for RNA virus-related diseases.

### Solution to Problem

The present invention includes the following aspects.
[1] A prophylactic or therapeutic agent for RNA virus-related diseases, including, as an active ingredient:
   at least one compound selected from the group consisting of a selective estrogen receptor modulator, an anti-tuberculosis drug, a CysLT1 receptor antagonist, a peroxisome proliferator-activated receptor γ (PPARy) agonist, an arachidonate 5-lipoxygenase (5-LOX) inhibitor, a derivative thereof, a pharmaceutically acceptable salt thereof, and a pharmaceutically acceptable solvate thereof.
[2] The prophylactic or therapeutic agent for RNA virus-related diseases according to [1], in which the selective estrogen receptor modulator is raloxifene, tamoxifen, toremifene, or clomifene.
[3] The prophylactic or therapeutic agent for RNA virus-related diseases according to [2], in which the tamoxifen is in a form of tamoxifen citrate.
[4] The prophylactic or therapeutic agent for RNA virus-related diseases according to [2], in which the toremifene is in a form of a toremifene citrate.
[5] The prophylactic or therapeutic agent for RNA virus-related diseases according to [1], in which the anti-tuberculosis drug is rifampin.
[6] The prophylactic or therapeutic agent for RNA virus-related diseases according to [1], in which the CysLT1 receptor antagonist is pranlukast.
[7] The prophylactic or therapeutic agent for RNA virus-related diseases according to [1], in which the PPARy agonist is pioglitazone.
[8] The prophylactic or therapeutic agent for RNA virus-related diseases according to [1], in which the 5-LOX inhibitor is zileuton.
[9] The prophylactic or therapeutic agent for RNA virus-related diseases according to any one of [1] to [8], in which the RNA virus is at least one kind of virus belonging to a family selected from the group consisting of Coronaviridae, Filoviridae, and Paramyxoviridae.
[10] The prophylactic or therapeutic agent for RNA virus-related diseases according to [9], in which the RNA virus is at least one kind of virus selected from the group consisting of SERS-CoV-2, Ebola virus, and Sendai virus.
[11] The prophylactic or therapeutic agent for RNA virus-related diseases according to any one of [1] to [10], in which the prophylactic or therapeutic agent contains raloxifene and pioglitazone, as active ingredients.
[12] The prophylactic or therapeutic agent for RNA virus-related diseases according to any one of [1] to [10], further including remdesivir, in which the prophylactic or therapeutic agent contains raloxifene and remdesivir, as active ingredients.
[13] The prophylactic or therapeutic agent for RNA virus-related diseases according to any one of [1] to [10], further including remdesivir, in which the prophylactic or therapeutic agent contains pioglitazone and remdesivir, as active ingredients.
[14] A prophylactic or therapeutic method for RNA virus-related diseases, including a step of administering an effective amount of the prophylactic or therapeutic agent for RNA virus-related diseases according to any one of [1] to [13] to a subject in need of the prophylactic or therapeutic agent.
[15] The prophylactic or therapeutic method for RNA virus-related diseases according to [14], further including a step of administering effective amounts of raloxifene and pioglitazone to a subject in need of raloxifene and pioglitazone.
[16] The prophylactic or therapeutic method for RNA virus-related diseases according to [14], further including a step of administering effective amounts of raloxifene and remdesivir to a subject in need of raloxifene and remdesivir.
[17] The prophylactic or therapeutic method for RNA virus-related diseases according to [14], further including a step of administering effective amounts of pioglitazone and remdesivir to a subject in need of pioglitazone and remdesivir.

### Advantageous Effects of Invention

According to the present invention, it is possible to provide a drug having a broad spectrum against an RNA virus.

### Brief Description of the Drawings

Fig. 1 is a schematic diagram showing the genomic structure of a Sendai virus (SeV) constructed in Experimental Example 1.
Fig. 2 is a photographic image showing the results of karyotype analysis of a human induced pluripotent stem cell (iPSC) prepared in Experimental Example 1.
Fig. 3 is a schematic view showing a timeline of a compound screening carried out in Experimental Example 1.
Fig. 4 is a schematic view showing an outline of the compound screening carried out in Experimental Example 1.
Fig. 5 is a graph showing the results of the first screening in Experimental Example 1.
Fig. 6 is a graph showing the results of the first screening in Experimental Example 1.
Fig. 7A is a graph showing result of quantitative RT-PCR in Experimental Example 2.
Fig. 7B is a graph showing result of quantitative RT-PCR in Experimental Example 2.
Fig. 8A is a graph showing the dose dependency of a drug on SeV infection, evaluated in Experimental Example 2.
Fig. 8B is a graph showing the dose dependency of a drug on SeV infection, evaluated in Experimental Example 2.
Fig. 8C is a graph showing the dose dependency of a drug on SeV infection, evaluated in Experimental Example 2.
Fig. 8D is a graph showing the dose dependency of a drug on SeV infection, evaluated in Experimental Example 2.
Fig. 8E is a graph showing the dose dependency of a drug on SeV infection, evaluated in Experimental Example 2.
Fig. 8F is a graph showing the dose dependency of a drug on SeV infection, evaluated in Experimental Example 2.
Fig. 9A is a graph showing the dose dependency of a drug on Ebola virus-like particle (Ebola trVLP) infection, evaluated in Experimental Example 3.
Fig. 9B is a graph showing the dose dependency of a drug on Ebola virus-like particle (Ebola trVLP) infection, evaluated in Experimental Example 3.
Fig. 9C is a graph showing the dose dependency of a drug on Ebola virus-like particle (Ebola trVLP) infection, evaluated in Experimental Example 3.
Fig. 9D is a graph showing the dose dependency of a drug on Ebola virus-like particle (Ebola trVLP) infection, evaluated in Experimental Example 3.
Fig. 9E is a graph showing the dose dependency of a drug on Ebola virus-like particle (Ebola trVLP) infection, evaluated in Experimental Example 3.
Fig. 9F is a graph showing the dose dependency of a drug on Ebola virus-like particle (Ebola trVLP) infection, evaluated in Experimental Example 3.
Fig. 10A is a graph showing the dose dependency of a drug on SARS-CoV-2 infection, evaluated in Experimental Example 4.
Fig. 10B is a graph showing the dose dependency of a drug on SARS-CoV-2 infection, evaluated in Experimental Example 4.
Fig. 10C is a graph showing the dose dependency of a drug on SARS-CoV-2 infection, evaluated in Experimental Example 4.
Fig. 10D is a graph showing the dose dependency of a drug on SARS-CoV-2 infection, evaluated in Experimental Example 4.
Fig. 10E is a graph showing the dose dependency of a drug on SARS-CoV-2 infection, evaluated in Experimental Example 4.
Fig. 10F is a graph showing the dose dependency of a drug on SARS-CoV-2 infection, evaluated in Experimental Example 4.
Fig. 11 is a dose response matrix and a three-dimensional synergy map for SARS-CoV-2 infection in a case of a combined use of raloxifene and remdesivir, evaluated in Experimental Example 4.
Fig. 12 is a dose response matrix and a three-dimensional synergy map for SARS-CoV-2 infection in a case of a combined use of raloxifene and pioglitazone, evaluated in Experimental Example 4.
Fig. 13 is a dose response matrix and a three-dimensional synergy map for SARS-CoV-2 infection in a case of a combined use of pioglitazone and remdesivir, evaluated in Experimental Example 4.
Fig. 14A is a graph showing the dose dependency of tamoxifen on Ebola trVLP infection, evaluated in Experimental Example 5.
Fig. 14B is a graph showing the dose dependency of toremifene on Ebola trVLP infection, evaluated in Experimental Example 5.
Fig. 14C is a graph showing the dose dependency of clomifene on Ebola trVLP infection, evaluated in Experimental Example 5.
Fig. 15A is a graph showing the dose dependency of tamoxifen on SARS-CoV-2 infection, evaluated in Experimental Example 5.
Fig. 15B is a graph showing the dose dependency of toremifene on SARS-CoV-2 infection, evaluated in Experimental Example 5.
Fig. 15C is a graph showing the dose dependency of clomifene on SARS-CoV-2 infection, evaluated in Experimental Example 5.
Fig. 16 is a schematic view showing the structure of a pseudo-type vesicular stomatitis virus (VSV) constructed in Experimental Example 6.
Fig. 17A is a graph showing the dose dependency of raloxifene on pseudo-type VSV infection, evaluated in Experimental Example 6.
Fig. 17B is a graph showing the dose dependency of toremifene on pseudo-type VSV infection, evaluated in Experimental Example 6.
Fig. 17C is a graph showing the dose dependency of clomifene on pseudo-type VSV infection, evaluated in Experimental Example 6.
Fig. 18 is a schematic view showing the mechanism (a part thereof) of the antiviral effect of the selective estrogen receptor modulator (SERM), shown in Experimental Example 6.

### Detailed Description of the Invention

### [Prophylactic or therapeutic agent for RNA virus-related diseases]

In one embodiment, the present invention provides a prophylactic or therapeutic agent for RNA virus-related diseases, including, as an active ingredient, at least one compound selected from the group consisting of a selective estrogen receptor modulator, an anti-tuberculosis drug, a CysLT1 receptor antagonist, a PPARy agonist, a 5-LOX inhibitor, a derivative thereof, a pharmaceutically acceptable salt thereof, and a pharmaceutically acceptable solvate thereof.

The prophylactic or therapeutic agent according to the present embodiment can prevent or treat RNA virus-related diseases by suppressing the infection of RNA virus into cells. Examples of the RNA virus-related diseases include diseases caused by the RNA virus infection, and specific examples thereof include a novel coronavirus infectious disease (COVID-19), severe acute respiratory syndrome (SARS), Middle East respiratory syndrome (MERS), common cold, Ebola hemorrhagic fever, Marburg disease, an infant respiratory tract infectious disease, mumps (epidemic parotid gland inflammation), an infant respiratory tract infectious disease, measles, subacute sclerosing panencephalitis, Hendra virus infectious disease, a Nipah virus infectious disease, Newcastle disease (conjunctivitis), and an infant upper respiratory tract infectious disease (cold).

As will be described below in the Examples, the inventors of the present invention constructed a compound screening system using the Sendai virus (SeV) and human induced pluripotent stem cells (iPSC) and screened a library of drugs approved by the U.S. Food and Drug Administration (FDA). As a result, compounds belonging to a selective estrogen receptor modulator, an anti-tuberculosis drug, a CysLT1 receptor antagonist, a PPARy agonist, and a 5-LOX inhibitor were identified as compounds that suppress SeV infection and have low cytotoxicity. Furthermore, the antiviral effect of the selected hit compound was evaluated with the Ebola virus (EBOV) and SARS-CoV-2, thereby completing the present invention.

SeV is a single-stranded negative sense RNA virus belonging to the genus Respirovirus of the subfamily Paramyxovirinae of Paramyxoviridae. SeV belongs to a mouse parainfluenza virus type 1, which has been found in Sendai, Japan, and it is naturally replicated in the respiratory mucous membrane. A recombinant attenuated form thereof has been constructed by genetic engineering, and SeV having a target gene is used for gene therapy in humans.

Human iPSCs are widely used in disease models and drug discovery, and undifferentiated iPSCs themselves are also useful for compound screening. Human iPSCs have an advantage in that they have human genes and have a normal karyotype and infinite self-renewal ability.

In the prophylactic or therapeutic agent according to the present embodiment, examples of the selective estrogen receptor modulator include raloxifene (CAS number: 84449-90-1), tamoxifen (CAS number: 10540-29-1), toremifene (CAS number: 89778-26-7), and clomifene (CAS number: 911-45-5).

The chemical formula of raloxifene is shown in Formula (1).

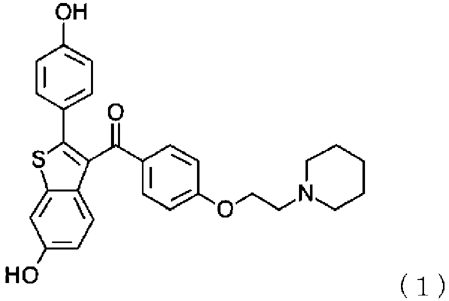

The chemical formula of tamoxifen is shown in Formula (2).

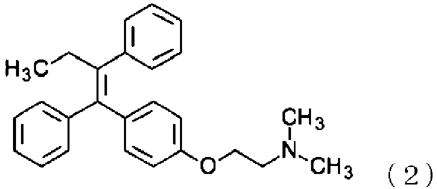

The chemical formula of toremifene is shown in Formula (3).

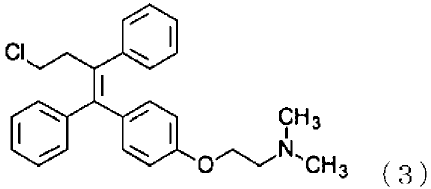

The chemical formula of clomifene is shown in Formula (4).

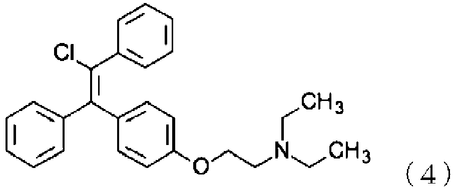

Tamoxifen is preferably in a form of tamoxifen citrate (CAS Number: 54965-24-1). The chemical formula of tamoxifen citrate is shown in Formula (5).

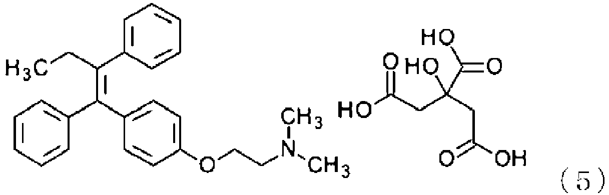

Toremifene is preferably in a form of toremifene citrate (CAS Number: 89778-27-8). The chemical formula of toremifene citrate is shown in Formula (6).

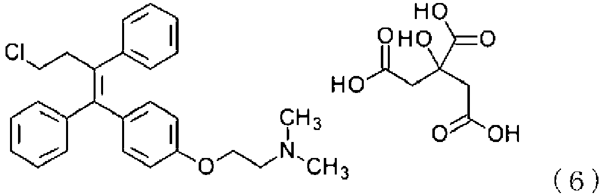

As will be described below in the Examples, the inventors of the present invention found that a selective estrogen receptor modulator inhibits the infection of SeV, EBOV, and SARS-CoV-2, which belong to families very different from each other across both the negative sense RNA virus and the positive sense RNA viruses. As a result, the selective estrogen receptor modulator is an example of RNA virus inhibitors having a broad spectrum. In addition, as will be described later in Examples, the inventors of the present invention have revealed that the selective estrogen receptor modulator inhibits the virus infection into host cells via a spike protein of the virus, thereby suppressing the RNA virus infection.

In the prophylactic or therapeutic agent according to the present embodiment, examples of the anti-tuberculosis drug include rifampin (CAS number: 13292-46-1). The chemical formula of rifampin is shown in Formula (7).

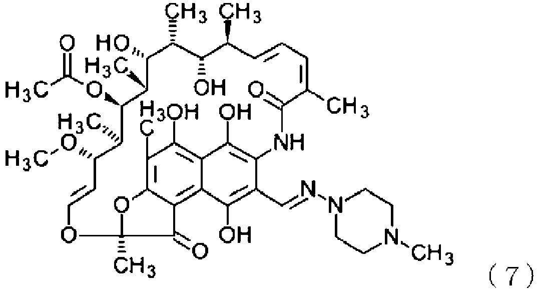

In the prophylactic or therapeutic agent according to the present embodiment, examples of the CysLT1 receptor antagonist include pranlukast (CAS Number: 103177-37-3). The chemical formula of pranlukast is shown in Formula (8).

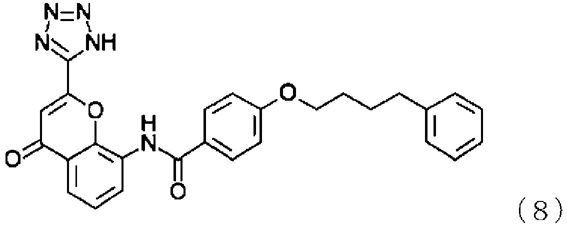

In the prophylactic or therapeutic agent according to the present embodiment, examples of the PPARy agonist include pioglitazone (CAS Number: 111025-46-8). The chemical formula of pioglitazone is shown in Formula (9).

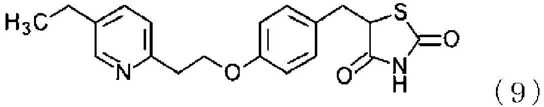

In the prophylactic or therapeutic agent according to the present embodiment, examples of the 5-LOX inhibitor include zileuton (CAS Number: 111406-87-2). The chemical formula of zileuton is shown in Formula (10).

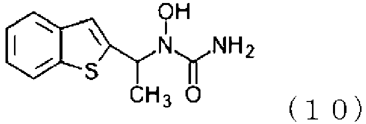

In the prophylactic or therapeutic agent according to the present embodiment, examples of the RNA virus include at least one kind of virus belonging to the family selected from the group consisting of Coronaviridae, Filoviridae, and Paramyxoviridae.

Examples of the virus belonging to Coronaviridae include severe acute respiratory syndrome coronavirus 2 (SERS-CoV-2). Examples of the virus belonging to Filoviridae include the Ebola virus. Examples of the virus belonging to Paramyxoviridae include the Sendai virus.

The prophylactic or therapeutic agent according to the present embodiment may contain raloxifene as the active ingredient. In addition, the prophylactic or therapeutic agent according to the present embodiment may further contain pioglitazone or remdesivir as the active ingredient, in addition to raloxifene.

That is, the prophylactic or therapeutic agent according to the present embodiment may contain raloxifene and pioglitazone as the active ingredient. As will be described below in the Examples, the inventors of the present invention have revealed that a combined use of raloxifene and pioglitazone synergistically suppresses RNA virus infection.

Alternatively, the prophylactic or therapeutic agent according to the present embodiment may contain raloxifene and remdesivir as the active ingredient. As will be described below in the Examples, the inventors of the present invention have revealed that a combined use of raloxifene and remdesivir synergistically suppresses RNA virus infection. The chemical formula of remdesivir (CAS number: 1809249-37-3) is shown in Formula (11).

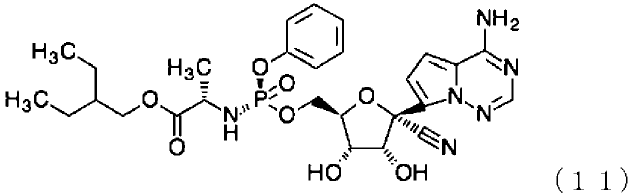

In addition, the prophylactic or therapeutic agent according to the present embodiment may contain pioglitazone and remdesivir as the active ingredient. As will be described below in the Examples, the inventors of the present invention have revealed that a combined use of pioglitazone and remdesivir synergistically suppresses RNA virus infection.

In the present specification, "containing as the active ingredient" means that it is contained as the main active ingredient and means that it is contained to the extent that the effect thereof is exhibited. The term "effect" in the present specification refers to a prophylactic or therapeutic effect on a targeted RNA virus-related disease, and in a certain aspect, may be an infection-suppressing effect on the targeted virus.

In the prophylactic or therapeutic agent according to the present embodiment, examples of the pharmaceutically acceptable salt include an inorganic acid salt, an alkali metal salt, an alkaline earth metal salt, a metal salt, an ammonium salt, an organic amine addition salt, and an amino acid addition salt. More specific examples thereof include inorganic acid salts such as a hydrochloride, a sulfate, a hydrobromide, a nitrate, and a phosphate; organic acid salts such as an acetate, a mesylate, a succinate, a maleate, a fumarate, a citrate, and a tartrate; alkali metal salts such as a sodium salt and a potassium salt; alkaline earth metal salts such as a magnesium salt and a calcium salt; metal salts such as an aluminum salt and a zinc salt; ammonium salts such as an ammonium salts and a tetramethylammonium salt; organic amine addition salts such as morpholine and piperidine; and amino acid addition salts of amino acids such as glycine, phenylalanine, lysine, aspartic acid, and glutamate. In addition, examples of the pharmaceutically acceptable solvate include a hydrate and an organic solvate.

It is preferable that the prophylactic or therapeutic agent according to the present embodiment be formulated as a pharmaceutical composition containing the above-described compound and a pharmaceutically acceptable carrier. The pharmaceutical composition can be administered orally in a form of, for example, a liquid agent, a powder agent, a granule agent, a tablet agent, or a capsule agent, or parenterally in a form of an injection agent, a suppository, or an external medicine for skin. Specific examples of the external medicine for skin include dosage forms such as an ointment and a patch.

As the pharmaceutically acceptable carrier, a carrier that is used for the pharmaceutical preparation of a general pharmaceutical composition can be used without particular limitation. More specific examples thereof include binders such as gelatin, cornstarch, gum tragacanth, and gum arabic; excipients such as starch and crystalline cellulose; swelling agents such as alginic acid; and solvents for an injection agent such as water, ethanol, and glycerin; and adhesives such as a rubber-based adhesive and a silicone-based adhesive.

The pharmaceutical composition may contain an additive. Examples of the additive include lubricants such as calcium stearate and magnesium stearate; sweetening agents such as sucrose, lactose, saccharin, and maltitol; flavoring agents such as peppermint and Akamono (Japanese azalea) oil; stabilizers such as benzyl alcohol and phenol; buffers such as a phosphate and sodium acetate; dissolution auxiliary agent such as benzyl benzoate and benzyl alcohol; antioxidants such as ascorbic acid; and preservatives such as parahydroxybenzoic acid esters (paraben), benzalkonium chloride, chlorobutanol, and cresol.

The dose administration of the pharmaceutical composition varies depending on the symptoms, body weight, age, gender, and the like of a patient and thus cannot be unconditionally determined. However, in a case of oral administration, for example, the active ingredient (the above-described compound) of 0.1 to 100 mg/kg body weight per administration unit form may be administered once or in 2 to 4 divided doses a day. Further, in the case of an injection agent, for example, the active ingredient of 0.01 to 50 mg per administration unit form may be administered.

### [Prophylactic or therapeutic method for RNA virus-related diseases]

In one embodiment, the present invention provides a prophylactic or therapeutic method for RNA virus-related diseases, including a step of administering an effective amount of the prophylactic or therapeutic agent for RNA virus-related diseases to a subject in need of the prophylactic or therapeutic agent.

In the present specification, the "effective amount" of a prophylactic or therapeutic agent can be said to be an agent containing an active ingredient (a compound) of an effective amount. Here, the "effective amount" of an agent or compound refers to an amount of the agent or compound required to bring about a prophylactic or therapeutic effect on the treated subject, and more specifically, it may be an amount sufficient for inhibiting, delaying, or minimizing at least one or more symptoms associated with the targeted RNA virus-related disease.

In a certain aspect, the "effective amount" of a compound may be an amount sufficient for inhibiting, delaying, or minimizing one or more symptoms associated with the targeted RNA virus-related disease either by the compound alone or in a combination with another compound or another treatment.

The effective amount of an agent or compound may vary depending on the targeted RNA virus-related disease, the administration route, the use of an excipient, and the combined use with another therapeutic treatment, as will be appreciated by those of skill in the art.

The prophylactic or therapeutic method according to the present embodiment may include a step of administering effective amounts of raloxifene and pioglitazone to a subject in need of raloxifene and pioglitazone.

Alternatively, the prophylactic or therapeutic method according to the present embodiment may include a step of administering effective amounts of raloxifene and remdesivir to a subject in need of raloxifene and remdesivir.

Alternatively, the prophylactic or therapeutic method according to the present embodiment may include a step of administering effective amounts of pioglitazone and remdesivir to a subject in need of pioglitazone and remdesivir.

### [Another embodiment]

In one embodiment, the present invention provides at least one compound for the prevention or treatment of RNA virus-related diseases, selected from the group consisting of a selective estrogen receptor modulator, an anti-tuberculosis drug, a CysLT1 receptor antagonist, a PPARy agonist, a 5-LOX inhibitor, a derivative thereof, a pharmaceutically acceptable salt thereof, and a pharmaceutically acceptable solvate thereof.

The selective estrogen receptor modulator, the anti-tuberculosis drug, the CysLT1 receptor antagonist, the PPARy agonist, the 5-LOX inhibitor, the derivative thereof, the pharmaceutically acceptable salt thereof, and the pharmaceutically acceptable solvate thereof are the same as those described above.

In one embodiment, the present invention provides a pharmaceutical composition for the prevention or treatment of RNA virus-related diseases, where the pharmaceutical composition contains raloxifene and pioglitazone as the active ingredient.

In one embodiment, the present invention provides a pharmaceutical composition for the prevention or treatment of RNA virus-related diseases, where the pharmaceutical composition contains raloxifene and remdesivir as the active ingredient.

In one embodiment, the present invention provides a pharmaceutical composition for the prevention or treatment of RNA virus-related diseases, where the pharmaceutical composition contains pioglitazone and remdesivir as the active ingredient.

In one embodiment, the present invention provides the use of at least one compound for the production of a prophylactic or therapeutic agent of RNA virus-related diseases, selected from the group consisting of a selective estrogen receptor modulator, an anti-tuberculosis drug, a CysLT1 receptor antagonist, a PPARy agonist, a 5-LOX inhibitor, a derivative thereof, a pharmaceutically acceptable salt thereof, and a pharmaceutically acceptable solvate thereof.

In one embodiment, the present invention provides the use of raloxifene and pioglitazone for the production of a prophylactic or therapeutic agent for RNA virus-related diseases.

In one embodiment, the present invention provides the use of raloxifene and remdesivir for the production of a prophylactic or therapeutic agent for RNA virus-related diseases.

In one embodiment, the present invention provides the use of pioglitazone and remdesivir for the production of a prophylactic or therapeutic agent for RNA virus-related diseases.

### Examples

Next, the present invention will be described in more detail by showing Examples; however, the present invention is not limited to Examples below.

### [Materials and methods]

### (Preparation of iPS cell)

A human iPSC was prepared from peripheral blood mononuclear cells (PBMC) using previously reported episomal vectors (Sox2, Klf4, Oct314, L-Myc, Lin28, and p53-shRNA) and cultured in a feeder-free culture system using StemFit (Ajinomoto Co., Inc.) culture medium. The karyotype analysis of the iPSC was carried out by LSI Medience Corporation.

### (Compound)

A library (Enzo Life Sciences Inc.) of drugs approved by the U.S. Food and Drug Administration (FDA) was used for high-throughput screening. The compounds used in the RNA quantification assay were purchased from Selleck Chemicals LLC.

### (Compound screening using the Sendai virus and human iPS cell)

For a high-throughput compound screening, human iPSC was dissociated into single cells with TrypLE Express (Thermo Fisher Scientific, Inc.) and seeded in a 96-well plate coated with iMatrix using StemFit containing 10 µM Y-27632 (Nacalai Tesque, Inc.). After 24 hours, the culture medium was replaced with a fresh StemFit containing a compound and incubated for 3 hours. Subsequently, the iPSC was exposed to the Sendai virus (SeV) having the EGFP gene. The multiplicity of infection (MOI) was estimated to be 1.

After incubating for 48 hours, cells were washed twice with PBS and subsequently fixed in 4% paraformaldehyde (PFA) at room temperature for 10 minutes. 4'6-Diamino-2-phenylindole (DAPI) (Thermo Fisher Scientific, Inc.) was used for the labeling of the nuclei. A cell image was acquired using IN CELL Analyzer 6000 (GE Healthcare) or IN CELL Analyzer 2000 (GE Healthcare), and the number of EGFP-positive cells was quantified by using IN CELL Developer Toolbox Software 1.92 (GE Healthcare).

### (Quantitative RT-PCR)

A human iPSC was seeded in an iMatrix-coated 24-well plate in StemFit (Ajinomoto Co., Inc.) and exposed to SeV. After washing the cells twice with PBS, the total RNA of the cultured human iPSC was extracted by using a miRNeasy Mini kit (QIAGEN N.V.). Subsequently, 500 ng of RNA was subjected to reverse transcription by using ReverTraAce (Toyobo Co., Ltd.). The mRNA level was measured by reverse transcription using oligo dT, and the viral genomic RNA level was measured by reverse transcription using random primers. The quantitative PCR analysis was carried out using SYBR Premix Ex Taq II (Takara Bio Inc.) and StepOnePlus system (Thermo Fisher Scientific, Inc.). PCR primers for detecting viral genomic RNA were designed at sites between the NP gene and the P gene. The base sequences of the primers used are shown in Table 1 below.

**[Table 1]**

| Primer name | Sequence (from 5' to 3') | Seq ID No. |
|---|---|---|
| EGFP_F | GGACGACGGCAACTACAAGA | 1 |
| EGFP_R | TTGTACTCCAGCTTGTGCCC | 2 |
| GAPDH_F | TCCACTGGCGTCTTCACC | 3 |
| GAPDH_R | GGCAGAGATGATGACCCTTTT | 4 |

### (Cell line)

293T cells, Huh7 cells, and Vero E6 cells (provided by Dr. Ayato Takada, Hokkaido University) were maintained in Dulbecco's modified Eagle's culture medium (DMEM) supplemented with 10% fetal bovine serum (FBS) and 1% penicillin/streptomycin solution.

### (Ebola trVLP production and infection assay)

In order to evaluate the anti-Ebola virus activity of a compound, Ebola trVLP expressing a GFP reporter was prepared using 293T cells (T. Hoenen, et. al., Modeling the lifecycle of Ebola virus under biosafety level 2 conditions with virus-like particles containing tetracistronic minigenomes. J Vis Exp, 52381, 2014). ApCAGGS expression plasmid encoding EBOV-NP, EBOV-VP35, EBOV-VP30, EBOV-L, and T7-polymerase, and an expression plasmid of the trVLP tetracistronic minigenome, encoding EBOV-GP, EBOV-VP40, EBOV-VP24, and a GFP reporter gene were transfected into 293T cells by using the calcium phosphate method. One day after transfection, the culture supernatant was replaced with a fresh culture medium. After 3 days, the supernatant was recovered and clarified by centrifugation at 2,000 × g for 15 minutes to obtain Ebola trVLP. The Ebola trVLP was stored at -80°C.

In order to increase the titer of the Ebola trVLP, the Ebola trVLP was passaged 2 or 3 times as follows. The pCAGGS expression plasmid encoding EBOV-NP, EBOV-VP35, EBOV-VP30, EBOV-L, and the host factor Tim-1 was transfected into 293T cells by using the calcium phosphate method. One day after transfection, the cells were exposed to trVLP for one day, cultured for 3 days, and then the culture supernatant was recovered to obtain Ebola trVLP with a high titer. In the experimental example of the present application, the Ebola trVLP with a high titer was used.

In order to assay the antiviral activity of the compound, a pCAGGS expression plasmid encoding EBOV-NP, EBOV-VP35, EBOV-VP30, and EBOV-L was transfected into Huh7 cells by using a TransIT LT1 transfection reagent (Minus Bio LLC). Two days after transfection, cells were pretreated with each compound having an appropriate concentration, exposed to the passaged trVLP, and then incubated in the presence of the compound for 2 days.

Subsequently, the cells exposed to trVLP were fixed overnight with 10% formalin, subsequently stained with a Hoechst 33342 dye for nuclear staining, and imaged with a Cytation 5 imaging plate reader (BioTek Instruments, Inc.) equipped with a 4× lens. Counting of cell nuclei and infected cells was carried out using Cell Profiler image analysis software (Broad Institute, USA) and a customized analysis pipeline.

### (Proliferation of SARS-CoV-2 and infection assay)

A SARS-CoV-2 strain (accession number: EPI-ISL-481251, GISAID) isolated from a Japanese patient was proliferated on Vero E6 cells. The culture supernatant was recovered 4 days after infection, clarified by centrifugation at 2,000 × g for 15 minutes, and stored at -80°C until use.

In order to assay the antiviral activity of the compound, Vero E6 cells were seeded in a 96-well plate and incubated for 1 hour in the presence of each compound having an appropriate concentration. Next, the cells were exposed to 0.002 of MOI of SARS-CoV-2 and incubated at 37°C in the presence of the compound. After 2 days, the cells were fixed overnight with 4% PFA.

The cells infected with SARS-CoV-2 were detected by fluorescent immunostaining using a rabbit anti-SARS-CoV N antibody as the primary antibody and an Alexa Fluor 488-labeled goat anti-rabbit IgG antibody as the secondary antibody. The total cell number was detected by nuclear staining with a Hoechst 33342 dye. The rate of the number of SARS-CoV-2 infected cells (the number of Alexa Fluor 488 positive cells) to the total number of cells (the number of Hoechst 33342 positive cells) was calculated as the SARS-CoV-2 infection rate (%).

The cells were imaged with a Cytation 5 imaging plate reader equipped with a 4× lens. Counting of cell nuclei and infected cells was carried out using CellProfiler image analysis software and a customized analysis pipeline. All experiments using the replicable SARS-CoV-2 were carried out in a biosafety level 3 (BSL3) laboratory at Nagasaki University, Japan.

### (Drug combination analysis)

To investigate the efficacy of the drug combination, SynergyFinder (https://synergyfinder.fimm.fi/) was used, and each combination of doses of raloxifene and remdesivir or pioglitazone was compared with those of each drug alone to calculate the inhibition rate (%) with respect to the virus infection. The synergy score for the combination of compounds was calculated using a Zero Interaction Potency (ZIP) model. It was determined that (i) in a case where the synergy score is less than -10, there is a high possibility that the interaction between two drugs is antagonistic, (ii) in a case where the synergy score is from -10 to 10, there is a high possibility that the interaction between two drugs is additive, and (iii) in a case where the synergy score is more than 10, there is a high possibility that the interaction between two drugs is synergistic.

### (Production of pseudo-type VSV and infection assay)

In order to analyze the mechanism of action of the drug, a vesicular stomatitis virus (VSV) which was mimicked each of the SARS-CoV-2 spike protein and the SARS-CoV spike protein and in which the VSV-G gene was replaced with a firefly luciferase reporter gene (may be each referred to as VSVΔG-SARS2-S and VSVΔG-SARS-S) were prepared.

A codon-optimized SARS-CoV-2 S gene having a C-terminal deletion of 19 amino acid residues and a codon-optimized SARS-CoV S gene having a C-terminal deletion of 19 amino acid residues were synthesized and inserted into a pCAGGS expression plasmid using In-Fusion HD (Clontech Laboratories, Inc.). The pCAGGS plasmid encoding the SARS-CoV-2 S gene or the SARS-CoV-S gene was transfected into 293T cells by the calcium phosphate method. One day after transfection, the cells were infected for 1 hour with recombinant VSV (hereinafter, may be referred to as VSVΔG-VSV-G) in which the VSV-G gene was replaced with a firefly luciferase reporter gene. One day after infection, the supernatant was collected, centrifuged at 2,000 × g for 15 minutes, and clarified to obtain VSVΔG-SARS2-S or VSVΔG-SARS-S. It was stored at -80°C until use.

As a control for the contamination of the infected virus, a VSV having the VSV glycoprotein (G) on the envelope (hereinafter, may be referred to as VSV-G) was produced. The pCAGGS plasmid encoding the G gene was transfected into 293T cells, exposed to VSVΔG-VSV-G for 1 hour, and after one day, the culture supernatant was collected to obtain VSV-G.

In order to evaluate the effect of a compound on the pseudo-type VSV infection, Vero E6 cells were seeded in a 96-well plate and incubated for 1 hour in the presence of each compound having an appropriate concentration. Subsequently, the cells were exposed to each pseudo-type VSV and incubated at 37°C in the presence of the compound. After 20 hours, the cells were lysed, and the luciferase activity was measured using a SpectraMax iD5 microplate reader (Molecular Devices, LLC).

### (Statistical analysis)

In order to determine statistical significance, the obtained results were subjected to the Dunnett's post-hoc test and subsequently analyzed using one-way ANOVA. A difference of p < 0.05 was regarded as significant. The analysis was carried out using GraphPad Prism software version 8.0 (GraphPad Software Inc.) for Windows.

### [Experimental Example 1]

### (Screening of anti-RNA virus drug by using human iPS cell and SeV)

A compound screening for identifying a therapeutic agent against RNA virus was carried out using the Sendai virus (SeV) and a human iPSC.

The SeV genome contains a 3' leader sequence and a 5' trailer sequence, and it encodes six genes that are transcribed in the order of nucleocapsid (N), phosphoprotein (P), matrix (M), fusion(F), hemagglutinin-neuraminidase (HN), and large polymerase (L).

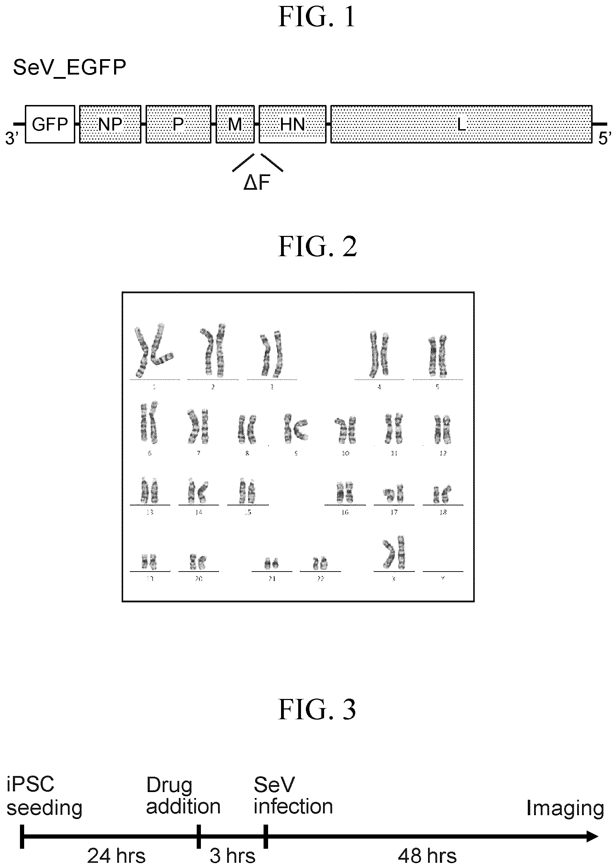

In order to eliminate the ability to produce progeny virus that could infect other cells, the F gene was removed to construct a SeV having an EGFP gene in the 3' region of the viral genomic RNA. Fig. 1 is a schematic diagram showing the genomic structure of the constructed SeV.

In addition, a human iPSC was prepared from a healthy subject. Fig. 2 is a photographic image showing the results of the karyotype analysis of the prepared iPSC. This iPSC was infected with SeV to construct an assay system for measuring viral replication by measuring the number of EGFP-positive iPSCs.

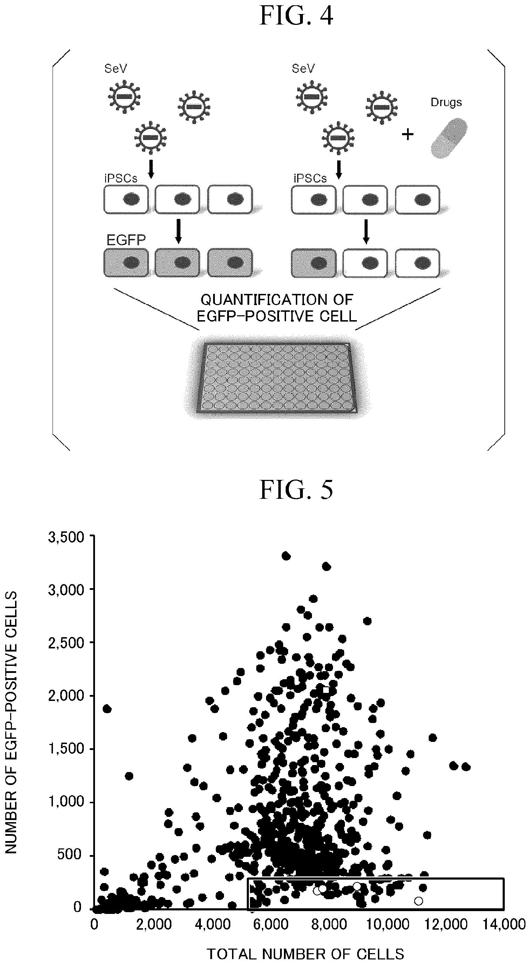

Fig. 3 is a schematic view showing a timeline of a compound screening. Fig. 4 is a schematic view showing an outline of the compound screening. The iPSC was seeded in a 96-well plate. After 24 hours, 10 µM of the compound was added thereto and incubated for 3 hours, and then the iPSC was exposed to SeV. The number of EGFP-positive cells was quantified 48 hours after the virus infection, and a compound that resulted in a decrease in the number of EGFP-positive cells compared with the negative subject (without compound addition) was extracted. In addition, the number of DAPI-positive cells (the total number of cells) was also quantified, and compounds that resulted in a significant decrease as compared with the negative subject were excluded as those having strong cytotoxicity. Then, the top 30 compounds having a small number of EGFP-positive cells were designated as hit compounds.

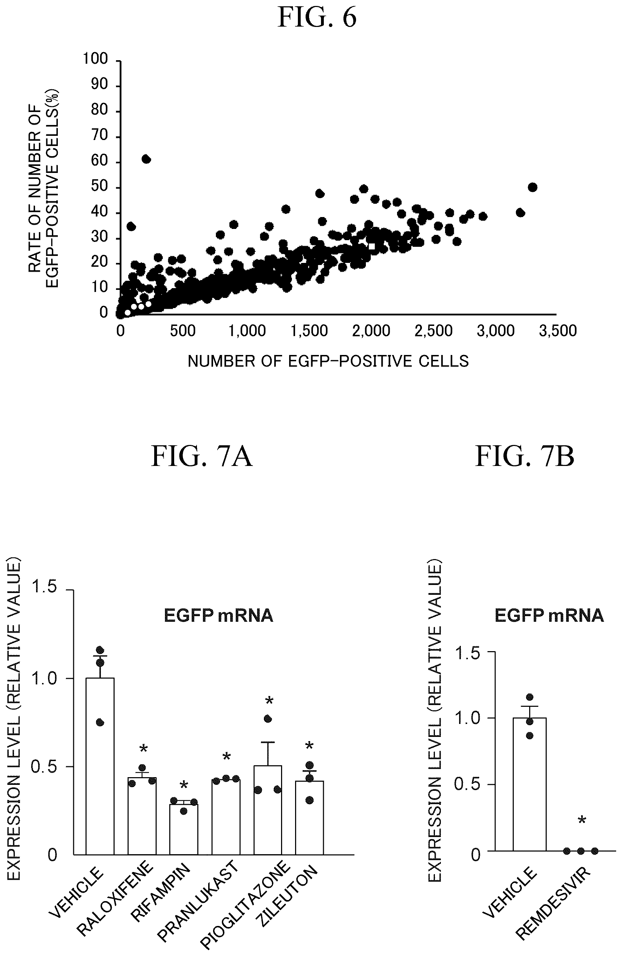

Fig. 5 and Fig. 6 are graphs showing the results of the first screening. About 500 compounds were evaluated. In Fig. 5 and Fig.6, white squares indicate negative control (DMSO). White circles indicate raloxifene, rifampin, pranlukast, zileuton, and pioglitazone among the hit compounds. Table 2 below shows a list of the hit compounds. In Table 2, the score indicates the number of EGFP-positive cells (a value subjected to correction between batches).

**[Table 2]**

| Number | Drug | Mechanism of action | Score |
|---|---|---|---|
| 1 | Raloxifene hydrochloride | ERa agonist/ERβ agonist | 57.97 |
| 2 | Dobutamine hydrochloride | β1 agonist/β2 agonist | 69.56 |
| 3 | Rifampin | RNA polymerase inhibitor | 75.36 |
| 4 | Chlorpromazine hydrochloride | DA antagonist | 75.36 |
| 5 | Aripiprazole | 5HT agonistID agonist | 86.95 |
| 6 | Sulindac | COX-1 inhibitor | 127.53 |
| 7 | Quetiapine fumarate | 5HT-2 antagonist/D2 antagonist | 133.33 |
| 8 | Felodipine | L-Ca²⁺ channel blocker | 133.33 |
| 9 | Domperidone | D2 antagonist/D3 antagonist | 139.13 |
| 10 | Zafirlukast | CycLT1 antagonist | 156.52 |
| 11 | Pranlukast | CycLT1 antagonist | 162.31 |
| 12 | Phenoxybenzamine hydrochloride | a antagonist | 162.31 |
| 13 | Pioglitazone | PPARγ agonist | 173.91 |
| 14 | Diazoxide | ATP>K activator | 173.91 |
| 15 | Pyrilamine maleate | H1 antagonist | 185.50 |
| 16 | Nicorandil | K⁺ channel activator | 185.50 |
| 17 | Maprotiline hydrochloride | NE incorporation inhibitor | 191.30 |
| 18 | Amitriptyline hydrochloride | SNRI | 192.61 |
| 19 | Cetirizine dihydrochloride | H1 agonist | 202.89 |
| 20 | Rofecoxib | COX-2 inhibitor | 202.89 |
| 21 | Glibenclamide | ATP>K blocker | 202.89 |
| 22 | Prostaglandin E2 | Prostaglandin | 208.69 |
| 23 | Celecoxib | COX-2 inhibitor | 208.69 |
| 24 | Ifenprodil tartrate | NMDA antagonist | 214.49 |
| 25 | Naftopidil | α1 antagonist | 214.49 |
| 26 | Zileuton | 5-LOX inhibitor | 214.49 |
| 27 | Thioridazine hydrochloride | D antagonist | 217.78 |
| 28 | (+)-Butaclamol hydrochloride | D antagonist | 226.08 |
| 29 | Minoxidil | ATP>K⁺ channel activator | 226.08 |
| 30 | Promethazine hydrochloride | H1 antagonist | 231.88 |

### [Experimental Example 2]

### (Inhibition of viral replication by FDA-approved drug)

From the hit compounds, drugs that have a small effect on cardiovascular circulation and the central nervous system were selected, and the next analysis was focused on the five drugs targeted to objects different from each other; raloxifene, which is a selective estrogen receptor modulator (SERM), rifampin, which is an anti-tuberculosis drug, pranlukast, which is a CysLT1 antagonist, pioglitazone, which is a PPARy agonist, and zileuton, which is a 5-LOX inhibitor.

An IPSC was seeded in a 24-well plate, incubated with 10 µM of each compound for 3 hours, and then infected with SeV. 24 hours after virus infection, RNA was extracted from the iPSC, and the amount of the EGFP mRNA was quantified using a quantitative RT-PCR method.

Fig. 7A shows the measurement results of the cell supplemented with raloxifene, rifampin, pranlukast, pioglitazone, or zileuton (all 10 µM, n = 3), and Fig. 7B shows the measurement results of the cell supplemented with remdesivir (1 µM, n = 3) approved by the FDA for application to COVID-19, which is an RNA-dependent RNA polymerase inhibitor. In Figs. 7A and 7B, "Vehicle" shows the result in the presence of DMSO, which is a negative control. In addition, the graph shows the average ± standard error, and "*" indicates that there is a significant difference at p < 0.005.

First, as shown in Fig. 7B, the EGFP mRNA was substantially not detected in the cell supplemented with remdesivir. This showed that the assay system using the iPSC and the SeV can detect the effect of the drug on the RNA-dependent RNA polymerase with good sensitivity. Next, from Fig. 7A, it was shown that the amount of the EGFP mRNA is also significantly and markedly reduced by the addition of raloxifene, rifampin, pranlukast, pioglitazone, or zileuton. This result shows that these hit compounds are capable of inhibiting the viral RNA synthesis activity or the viral life cycle step upstream thereof.

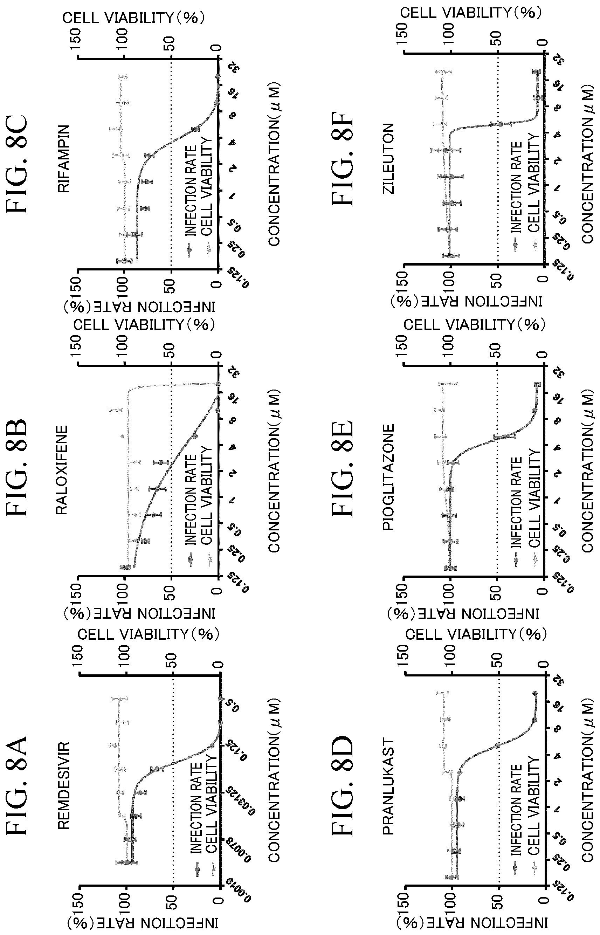

Subsequently, the dose dependency of these drugs on SeV infection was evaluated. Figs. 8A to 8F are each a graph showing the dose dependency of remdesivir, raloxifene, rifampin, pranlukast, pioglitazone, and zileuton.

As a result, remdesivir reduced the infection rate of SeV at an IC₅₀ value of 0.077 µM. This showed that this assay system is useful as an evaluation system for the inhibitory effect of a drug on RNA virus infection. In addition, raloxifene, rifampin, pranlukast, pioglitazone, and zileuton each showed IC₅₀ values of 4.3 µM, 3.9 µM, 5.0 µM, 4.9 µM, and 4.5 µM, and thus it was revealed that they reduce the infection rate of SeV in a dose-dependent manner.

As a result, it was shown that similar to remdesivir, raloxifene, rifampin, pranlukast, pioglitazone, and zileuton can effectively suppress the infection of cells with Sendai virus, which is one of the RNA viruses.

### [Experimental Example 3]

### (Evaluation of compound using Ebola virus life cycle modeling system)

In order to assay the antiviral activity of the selected compound, the anti-Ebola virus activity was evaluated by using a transcriptional and replicable virus-like particle (trVLP) system that does not require a biosafety level 4 (BSL4) laboratory, unlike a case of wild-type viruses.

Huh7 cells derived from the human liver were exposed to Ebola trVLP expressing a GFP reporter in the presence of each compound having various concentrations, and the number of infected cells and cell viability were measured.

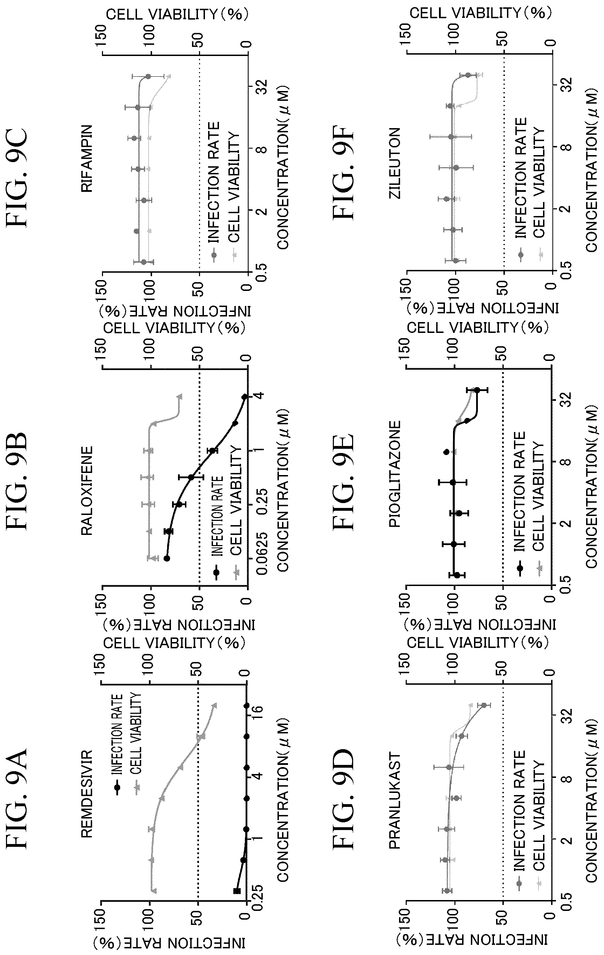

Figs. 9A to 9F are each a graph showing the dose dependency of remdesivir, raloxifene, rifampin, pranlukast, pioglitazone, and zileuton. As a result, it was shown that remdesivir, which is a nucleotide analog developed as a therapeutic agent for an Ebola virus infectious disease, strongly inhibits the Ebola trVLP infection, and the IC₅₀ value is 0.12 µM, demonstrating the validity of this assay system.

In addition, raloxifene also reduced the infection rate of Ebola trVLP in a dose-dependent manner, and the IC₅₀ value was 0.88 µM. Furthermore, it was shown that a specific antiviral effect is exhibited since a significant dissociation between the infection-inhibiting activity and the cytotoxicity was observed. In contrast, rifampin, pranlukast, pioglitazone, and zileuton did not show significant antiviral activity.

From the above results, it was shown that raloxifene is capable of being a prophylactic or therapeutic agent for an Ebola virus infectious disease.

### [Experimental Example 4]

### (Evaluation of compound against SARS-CoV-2)

The antiviral activity of the selected compound was evaluated by using SARS-CoV-2. Vero E6 cells were exposed to the wild-type SARS-CoV-2 in the presence of each compound having various concentrations, and then the number of infected cells was quantified using an immunostaining method to determine cell viability.

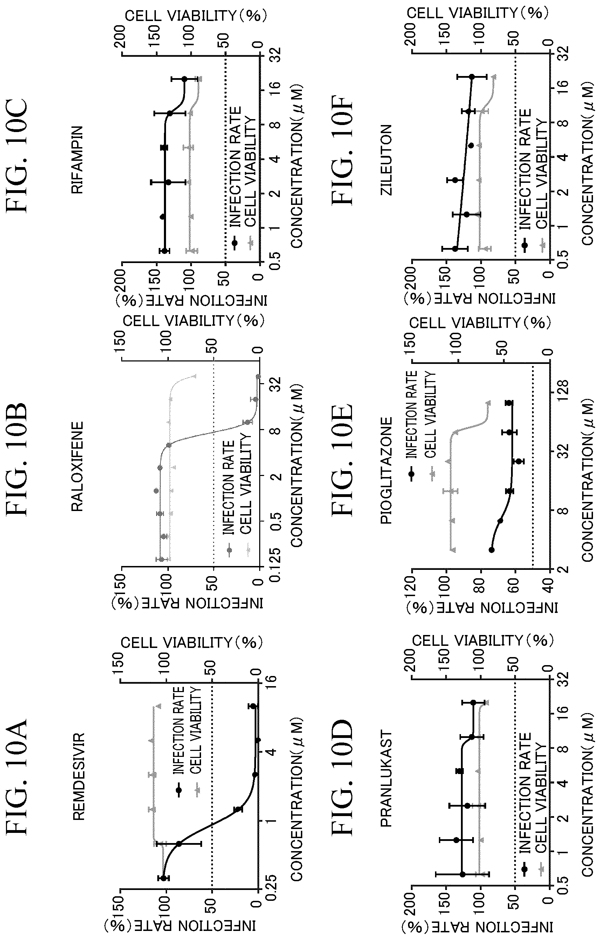

Figs. 10A to 10F are each a graph showing the dose dependency of remdesivir, raloxifene, rifampin, pranlukast, pioglitazone, and zileuton. As a result, remdesivir, which is approved by the FDA for application to COVID-19, reduced the infection rate of SARS-CoV-2 at an IC₅₀ value of 0.89 µM.

As shown in Fig. 10B, raloxifene reduced the infection rate of SARS-CoV-2 in a dose-dependent manner at an IC₅₀ value of 7.1 µM. In addition, pioglitazone partially suppressed virus infection at a high concentration thereof. In contrast, although it was observed that rifampin and pranlukast tended to reduce the infection rate of SARS-CoV-2, which was not significant, zileuton did not exhibit significant antiviral activity.

From the above results, it was shown that raloxifene and pioglitazone are capable of being a prophylactic or therapeutic agent for a SARS-CoV-2 infectious disease.

Subsequently, using SynergyFinder, the combined effects of raloxifene and remdesivir, raloxifene and pioglitazone, and pioglitazone and remdesivir were examined (Ianevski A, Giri AK and Aittokallio T (2020), SynergyFinder 2.0: visual analytics of multi-drug combination synergies, Nucleic Acids Res 48, W488-W493, 2020).

Regarding each of the two-compound combinations of raloxifene and remdesivir, raloxifene and pioglitazone, and pioglitazone and remdesivir, Vero E6 cells were exposed to the wild-type SARS-CoV-2 in the presence of each compound having various concentrations, the number of infected cells was subsequently quantified using an immunostaining method to determine cell viability, and the combined effect was examined.

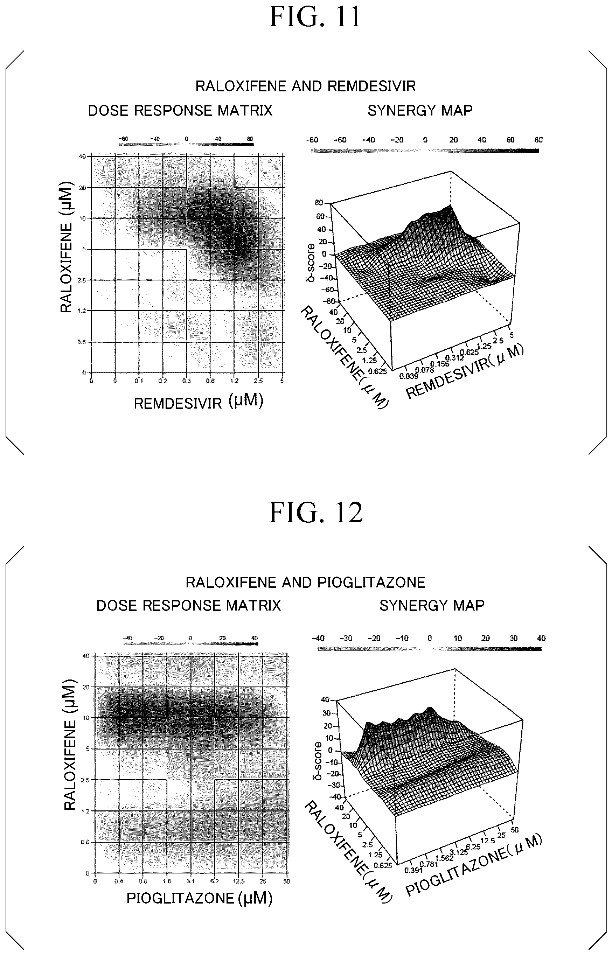

Fig. 11 is a dose response matrix and a three-dimensional synergy map for the combined use of raloxifene and remdesivir. It was indicated that (i) in a case where the synergy score is less than -10, there is a high possibility that the interaction between two drugs is antagonistic, (ii) in a case where the synergy score is from -10 to 10, there is a high possibility that the interaction between two drugs is additive, and (iii) in a case where the synergy score is more than 10, there is a high possibility that the interaction between two drugs is synergistic.

As a result, it was revealed that raloxifene and remdesivir exhibit a synergistic antiviral effect at a specific concentration. The ZIP synergy score was 6.91, and the highest synergy score was 30.09.

Fig. 12 is a dose response matrix and a three-dimensional synergy map for the combined use of raloxifene and pioglitazone. As a result, it was revealed that raloxifene and pioglitazone also exhibit a synergistic antiviral effect at a specific concentration. The ZIP synergy score was 4.49, and the highest synergy score was 12.42.

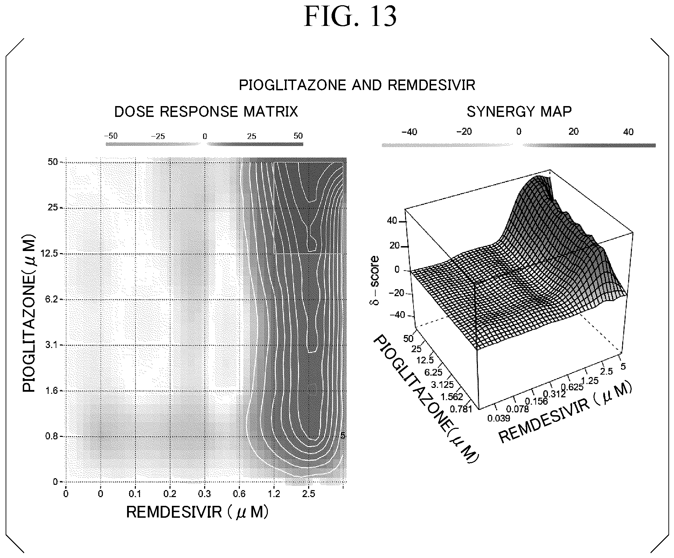

Fig. 13 is a dose response matrix and a three-dimensional synergy map for the combined use of pioglitazone and remdesivir. As a result, it was revealed that pioglitazone and remdesivir also exhibit a synergistic antiviral effect at a specific concentration. The ZIP synergy score was 8.85, and the highest synergy score was 30.42.

From the above results, it was strongly suggested that in a case where raloxifene and remdesivir, raloxifene and pioglitazone, or pioglitazone and remdesivir are used in combination, a synergistic effect is exhibited, and thus a very high antiviral effect is exhibited.

### [Experimental Example 5]

### (Effect of SERM on SARS-CoV-2 infection)

As described above, among the selected compounds, raloxifene exhibited antiviral activity against both the Ebola virus and the SARS-CoV-2. Raloxifene is a cancer treatment drug approved by the FDA and is a selective estrogen receptor modulator (SERM).

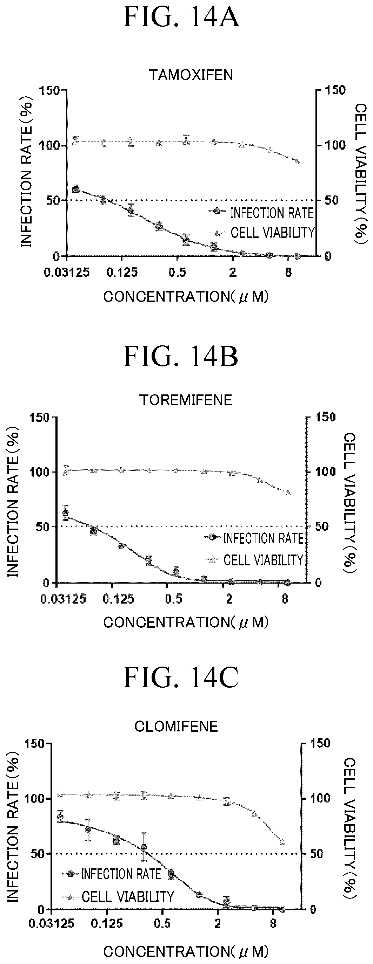

Accordingly, first, it was examined whether or not tamoxifen, toremifene, and clomifene, which are SERMs other than raloxifene, inhibit the Ebola trVLP infection by using the trVLP system. Huh7 cells derived from the human liver were exposed to Ebola trVLP expressing a GFP reporter in the presence of each compound having various concentrations, and the number of infected cells and cell viability were measured. Figs. 14A to 14C are each graphs showing the dose dependency of tamoxifen, toremifene, and clomifene. As a result, it was confirmed that tamoxifen, toremifene, and clomifene all inhibit the Ebola trVLP infection.

Subsequently, the antiviral activity of tamoxifen, toremifene, and clomifene was evaluated by using SARS-CoV-2. Vero E6 cells were exposed to the wild-type SARS-CoV-2 in the presence of each compound having various concentrations, and then the number of infected cells was quantified using an immunostaining method to determine cell viability.

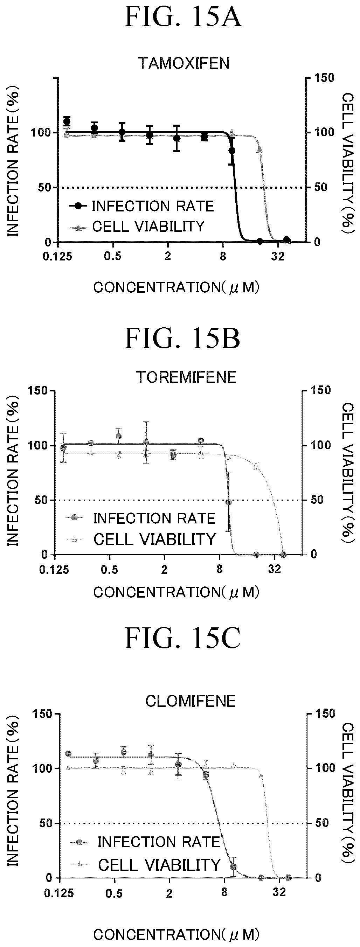

Figs. 15A to 15C are each graphs showing the dose dependency of tamoxifen, toremifene, and clomifene. As a result, it was revealed that tamoxifen, toremifene, and clomifene each exhibit an effect of suppressing the SARS-CoV-2 infection in Vero E6 cells at IC₅₀ values of 10.9 µM, 10.0 µM, and 6.7 µM, respectively.

From the above results, it was shown that raloxifene, tamoxifen, toremifene, and clomifene are all capable of being a prophylactic or therapeutic agent for a SARS-CoV-2 infectious disease. Furthermore, it was strongly suggested that SERM is capable of being a prophylactic or therapeutic agent for a SARS-CoV-2 infectious disease.

### [Experimental Example 6]

### (Analysis of mechanism of antiviral effect of SERM)

The mechanism of the antiviral effect of SERM was analyzed. In the coronavirus such as SARS-CoV-2, a viral core containing a viral genomic RNA is enclosed by an envelope (an outer membrane) consisting of a lipid bilayer and an outer membrane protein. In SARS-CoV-2, a spike protein, which is one of the outer membrane proteins, binds to a receptor on the cell membrane of the host cell, and then infection is established by 1) after the spike protein invades the inside of the cell by endocytosis, the envelope and the endosome membrane are fused, and then the virus genome is released in the inside of the cell, or 2) the spike protein is activated by a host protease present on the cell membrane, and the envelope and the cell membrane are fused, thereby releasing the viral genome in the inside of the cell.

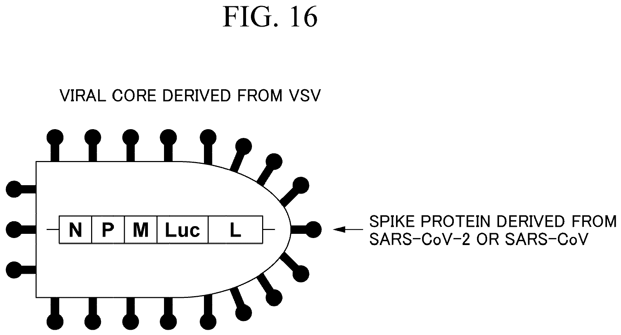

Accordingly, a pseudo-type vesicular stomatitis virus (VSVΔG-SARS2-S) having a luciferase gene and further having the spike protein of SARS-CoV-2 as a spike protein or a pseudo-type vesicular stomatitis virus (VSVΔG-SARS-S) having a luciferase gene and further having the spike protein of SARS-CoV as a spike protein was constructed (Fig. 16), and it was examined whether SERM inhibits the invasion of the pseudo-type VSV into the host cell. In addition, for comparison, a similar examination was carried out using a vesicular stomatitis virus (VSV-G) having a luciferase gene and the VSV glycoprotein (G) on the envelope.

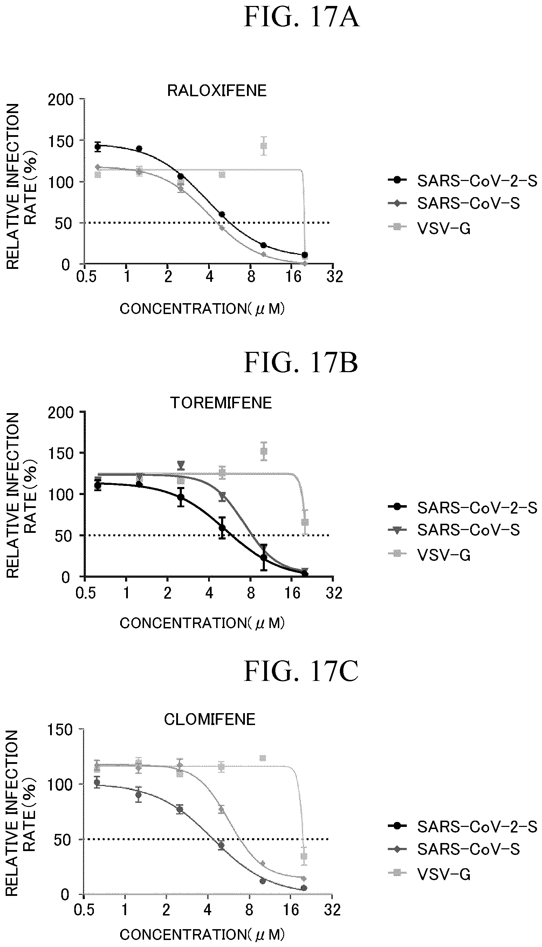

Figs. 17A to 17C show the results of incubating Vero E6 cells for 1 hour in the presence of raloxifene, toremifene, or clomifene, exposing them to the pseudo-type VSV, and measuring the luciferase activity after cytolysis. The lateral axis indicates the treatment concentration of each drug, and the vertical axis indicates the relative value relative to the luciferase activity obtained in the drug-untreated group. As shown in Figs. 17A to 17C, raloxifene, toremifene, and clomifene each inhibited the VSVΔG-SARS2-S infection at IC₅₀ values of 3.9 µM, 5.3 µM, and 4.4 µM, respectively. In addition, raloxifene, toremifene, and clomifene each also inhibited the infection with a pseudo-typed VSV having a SARS-CoV spike protein (SARS-CoV-S) at IC₅₀ values of 4.1 µM, 7.3 µM, and 5.8 µM, respectively. However, no infection-inhibiting effect was observed on VSV-G.

As a result, it was shown that although none of raloxifene, toremifene, or clomifene suppresses the invasion of the original vesicular stomatitis virus into the host cell, this drug effectively suppresses the invasion into the host cell in a case where the spike protein of SARS-CoV-2 or SARS-CoV-2 is expressed on the envelope of the virus.

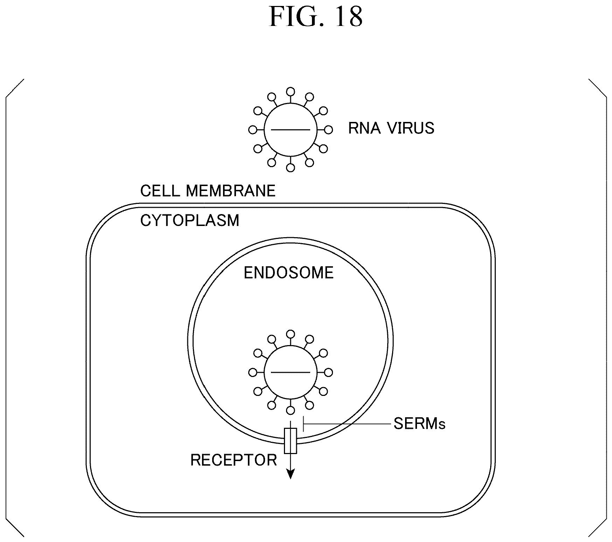

From the above results, it was suggested that SERM exhibits an antiviral effect by suppressing the invasion of a pathogenic coronavirus into the host cell via the virus spike protein. Fig. 18 is a schematic view showing the mechanism (a part thereof) of the antiviral effect of SERM. It is possible to conceive that SERM disrupts ion homeostasis in endosomes containing viral particles and inhibits membrane fusion for releasing the viral core into the cytoplasm of the host cell.

Table 3 below summarizes the effects of each of the compounds on viruses. In Table 3, "+" indicates that virus infection was suppressed, "-" indicates that virus infection was not suppressed, "N.S." (Not Significant) indicates that although a tendency to suppress infection was observed, it was not significant, and "N.D." (Not Determined) indicates that the test was not carried out.

**[Table 3]**

| Virus | | Compound | | | | | | | |
|---|---|---|---|---|---|---|---|---|---|
| | | Raloxifene | Rifampin | Pranlukast | Zileuton | Pioglitazone | Tamoxifen | Toremifene | Clomifene |
| Single-stranded positive sense RNA virus | | | | | | | | | |
| Coronaviridae | SARS-CoV | N.D. | N.D. | N.D. | N.D. | N.D. | N.D. | N.D. | N.D. |
| | SARS-CoV-2 | + | N.S. | N.S. | - | + | + | + | + |
| | MERS-CoV | N.D. | N.D. | N.D. | N.D. | N.D. | N.D. | N.D. | N.D. |
| Flaviviridae | Dengue virus | N.D. | N.D. | N.D. | N.D. | N.D. | N.D. | N.D. | N.D. |
| | Zika virus | N.D. | N.D. | N.D. | N.D. | N.D. | N.D. | N.D. | N.D. |
| | Hepatitis C virus | N.D. | N.D. | N.D. | N.D. | N.D. | N.D. | N.D. | N.D. |

| Single-stranded negative sense RNA virus | | | | | | | | | |
|---|---|---|---|---|---|---|---|---|---|
| Paramyxoviridae | Sendai virus | + | + | + | + | + | N.D. | N.D. | N.D. |
| Filoviridae | Ebola virus | + | - | - | - | - | + | + | + |
| Orthomyxoviridae | Influenza virus | N.D. | N.D. | N.D. | N.D. | N.D. | N.D. | N.D. | N.D. |

### Industrial Applicability

According to the present invention, it is possible to provide a prophylactic or therapeutic agent for RNA virus-related diseases.

## Claims

1. A prophylactic or therapeutic agent for RNA virus-related diseases, comprising, as an active ingredient:
at least one compound selected from the group consisting of a selective estrogen receptor modulator, an anti-tuberculosis drug, a CysLT1 receptor antagonist, a peroxisome proliferator-activated receptor γ (PPARy) agonist, an arachidonate 5-lipoxygenase (5-LOX) inhibitor, a derivative thereof, a pharmaceutically acceptable salt thereof, and a pharmaceutically acceptable solvate thereof.

2. The prophylactic or therapeutic agent for RNA virus-related diseases according to claim 1,
wherein the selective estrogen receptor modulator is raloxifene, tamoxifen, toremifene, or clomifene.

3. The prophylactic or therapeutic agent for RNA virus-related diseases according to claim 2,
wherein the tamoxifen is in a form of tamoxifen citrate.

4. The prophylactic or therapeutic agent for RNA virus-related diseases according to claim 2,
wherein the toremifene is in a form of a toremifene citrate.

5. The prophylactic or therapeutic agent for RNA virus-related diseases according to claim 1,
wherein the anti-tuberculosis drug is rifampin.

6. The prophylactic or therapeutic agent for RNA virus-related diseases according to claim 1,
wherein the CysLT1 receptor antagonist is pranlukast.

7. The prophylactic or therapeutic agent for RNA virus-related diseases according to claim 1,
wherein the PPARγ agonist is pioglitazone.

8. The prophylactic or therapeutic agent for RNA virus-related diseases according to claim 1,
wherein the 5-LOX inhibitor is zileuton.

9. The prophylactic or therapeutic agent for RNA virus-related diseases according to any one of claims 1 to 8,
wherein the RNA virus is at least one kind of virus belonging to a family selected from the group consisting of Coronaviridae, Filoviridae, and Paramyxoviridae.

10. The prophylactic or therapeutic agent for RNA virus-related diseases according to claim 9,
wherein the RNA virus is at least one kind of virus selected from the group consisting of severe acute respiratory syndrome coronavirus 2 (SERS-CoV-2), Ebola virus, and Sendai virus.

11. The prophylactic or therapeutic agent for RNA virus-related diseases according to any one of claims 1 to 10,
wherein the prophylactic or therapeutic agent contains raloxifene and pioglitazone, as active ingredients.

12. The prophylactic or therapeutic agent for RNA virus-related diseases according to any one of claims 1 to 10, further comprising:
remdesivir,
wherein the prophylactic or therapeutic agent contains raloxifene and remdesivir, as active ingredients.

13. The prophylactic or therapeutic agent for RNA virus-related diseases according to any one of claims 1 to 10, further comprising:
remdesivir,
wherein the prophylactic or therapeutic agent contains pioglitazone and remdesivir, as active ingredients.
